# EUROPÄISCHE PATENTSCHRIFT

(11) **EP 2 666 444 B1**
(45) Veröffentlichungstag und Bekanntmachung des Hinweises auf die Patenterteilung: **20.12.2017**
(21) Anmeldenummer: 13167654.6
(22) Anmeldetag: 14.05.2013
(51) Int. Cl.: A61F 2/966

(54) **Zuführsystem für ein medizinisches Funktionselement, Set mit einem derartigen Zuführsystem und Verfahren zum Entlassen eines medizinischen Funktionselements aus einem derartigen Zuführsystem**
Feed system for a medical functional element, kit including such a feed system and a method for releasing a medical functional element from such a feed system
Système d'acheminement pour un élément fonctionnel médical, kit comprenant un tel système d'acheminement et procédé de vidange d'un élément fonctionnel médical constitué d'un tel système d'acheminement

(30) Priorität: 25.05.2012 DE 102012104544
(43) Veröffentlichungstag der Anmeldung: 27.11.2013
(73) Patentinhaber: Acandis GmbH & Co. KG, 76327 Pfinztal (DE)
(72) Erfinder: Cattaneo, Giorgio, 76199 Karlsruhe (DE)
(74) Vertreter: Kilchert, Jochen

(56) Entgegenhaltungen:
- WO-A2-2012/068175
- DE-A1-102009 056 448
- DE-A1-102010 050 569
- US-A1- 2005 288 763
- US-A1- 2008 132 989

## Beschreibung

Die Erfindung betrifft ein Zuführsystem für ein medizinisches Funktionselement gemäß dem Oberbegriff des Patentanspruchs 1. Ferner betrifft die Erfindung ein Set mit einem derartigen Zuführsystem und einem medizinischen Funktionselement sowie ein Verfahren zum Entlassen eines medizinischen Funktionselements aus einem derartigen Zuführsystem. Ein Zuführsystem der eingangs genannten Art ist beispielsweise aus EP 1 374 801 A1 oder aus US 2008/132989 A1 bekannt.

Im Allgemeinen werden bekannte Zuführsysteme dazu genutzt, Stents an ihren Bestimmungsort zu führen. Das bekannte Zuführsystem nutzt dazu einen Katheter, in dem ein Führungsdraht längsverschieblich angeordnet ist. Am distalen Ende des Führungsdrahts sind zwei voneinander angeordnete Coils vorgesehen, die einen Aufnahmebereich für einen Stent begrenzen. Der zu implantierende Stent ist also zwischen den beiden Coils angeordnet. Der Stent weist einen komprimierten und einen expandierten Zustand auf, wobei der Stent durch die radiale Begrenzung innerhalb des Katheters den komprimierten Zustand einnimmt. Durch eine Relativbewegung zwischen dem Katheter und dem Führungsdraht wird das distale Ende des Führungsdrahts einschließlich des Aufnahmebereichs für den Stent aus dem Katheter entlassen. Infolgedessen entfällt die radiale Begrenzung, so dass der Stent in den expandierten Zustand übergeht.

Die Expansion des Stents erfolgt bei der Implantation in gerade Blutgefäße im Wesentlichen gleichmäßig zu allen Radialrichtungen. Der Stent tendiert also dazu, die Form eines Kreiszylinders anzunehmen. Eine solche gleichmäßige Aufweitung des Stents ist jedoch bei der Implantation in gekrümmte Blutgefäßabschnitte nicht immer gewährleistet. Insbesondere in gekrümmten Gefäßabschnitten mit engem Krümmungsradius besteht die Gefahr, dass der Stent kollabiert oder zumindest eine im Wesentlichen ovale Querschnittskontur einnimmt. Dadurch wird die eigentliche Funktion des Stents, nämlich das Wiedereröffnen eines Strömungskanals, nicht erfüllt. Vielmehr kann das Einknicken des Stents dazu führen, dass der Blutfluss durch das Gefäß weiter reduziert wird.

Die Aufgabe der Erfindung besteht darin, ein Zuführelement anzugeben, das ein zuverlässiges Aufweiten des Stents auch in gekrümmten Gefäßabschnitten gewährleistet. Ferner besteht die Aufgabe der Erfindung darin, ein Set mit einem derartigen Zuführsystem und einem Funktionselement und ein Verfahren zum Entlassen eines Funktionselements aus einem Zuführsystem anzugeben. Erfindungsgemäß wird diese Aufgabe im Hinblick auf das Zuführsystem durch den Gegenstand des Patentanspruchs 1, im Hinblick auf das Set durch den Gegenstand des Patentanspruchs 6 und im Hinblick auf das Verfahren durch den Gegenstand des Patentanspruchs 7 gelöst.

Die Erfindung beruht insbesondere auf dem Gedanken, ein Zuführsystem für ein medizinisches Funktionselement mit einem Katheter und einem Betätigungselement anzugeben, das im Katheter längsverschieblich angeordnet ist und in einem distalen Endabschnitt einen Aufnahmebereich für das Funktionselement aufweist. Das Betätigungselement ist mit dem Funktionselement lösbar verbindbar, so dass das Funktionselement im Gebrauch durch das Betätigungselement relativ zum Katheter bewegbar ist. Im Aufnahmebereich kann ein Expansionshilfselement angeordnet sein, das innerhalb des Katheters radial vorgespannt ist. Das Expansionshilfselement kann sich nach der Entlassung aus dem Katheter selbsttätig aufweiten. Dabei weist das Expansionshilfselement eine radiale Expansionshilfskraft auf, so dass eine radiale Aufweitung des Funktionselements unterstützbar ist oder unterstützt wird.

Erfindungsgemäß ist das Expansionshilfselement im Aufnahmebereich angeordnet. Somit ist sichergestellt, dass die radiale Expansionshilfskraft des Expansionshilfselements auf das im Aufnahmebereich angeordnete Funktionselement wirkt. Das radial vorgespannte bzw. selbstexpandierbare Expansionshilfselement drückt also nach Entlassung aus dem Katheter gegen eine Innenwand des Funktionselements. Somit wird die radiale Aufweitung des Funktionselements zumindest in einer ersten Expansionsphase wirksam unterstützt. Das Funktionselement kann ebenfalls selbstexpandierbar sein. Zusätzlich zu der Radialkraft, des selbstexpandierbaren Funktionselements wirkt die Expansionshilfskraft des Expansionshilfselements. Somit wird ein Kollabieren des Funktionselements beim Einsatz in gekrümmten Gefäßabschnitten effizient vermieden.

Bei einer bevorzugten Ausführungsform des erfindungsgemäßen Zuführsystems weist das Expansionshilfselement ein proximales Ende und ein distales Ende auf. Das proximale Ende kann fest mit dem Betätigungselement verbunden sein. Das distale Ende ist vorzugsweise längsverschieblich mit dem Betätigungselement verbunden bzw. auf dem Betätigungselement angeordnet. Durch die Längsbeweglichkeit des distalen Endes des Expansionshilfselements und die gleichzeitige fixierte Anordnung des proximalen Endes des Expansionshilfselements, wird erreicht, dass die Länge des Expansionshilfselements variabel ist. Somit kann die Längserstreckung des Expansionshilfselements ein Reservoir für die radiale Aufweitung bilden. Das bedeutet konkret, dass sich das Expansionshilfselement im komprimierten Zustand, der insbesondere innerhalb des Katheters vorliegt, eine relativ große Länge aufweist, also gestreckt ist. Sobald das Expansionshilfselement den Katheter verlässt und somit die radiale Begrenzung entfällt, bewirkt die radiale Expansionshilfskraft des Expansionshilfselements, dass sich das Expansionshilfselement radial aufweitet. Dabei verkürzt sich das Expansionshilfselement, wobei die Längendifferenz zwischen dem gestreckten Zustand und dem verkürzten Zustand des Expansionshilfselements im Wesentlichen ein Reservoir für die radiale Expansion des Expansionshilfselements bietet.

Indem das proximale Ende am Betätigungselement festgelegt und das distale Ende des Expansionshilfselements beweglich ist, wird außerdem erreicht, dass sich das Expansionshilfselement von distal nach proximal aufweitet. Die Aufweitung des Expansionshilfselements erfolgt also in derselben Richtung bzw. Abfolge wie die Expansion des Funktionselements. Da sich das Expansionshilfselement bei der radialen Aufweitung verkürzt, wandert der Bereich des Expansionshilfselements, der die Expansionshilfskraft auf das Funktionselement ausübt, entlang des Funktionselements von distal nach proximal. Insbesondere ist die Expansionshilfskraft in dem Bereich des Expansionshilfselements wirksam, der unmittelbar benachbart zur Katheterspitze aufgeweitet wird. Somit wird die Expansion des Funktionselements unmittelbar nach Entlassung aus dem Katheter bewirkt, insbesondere abschnittsweise entlang des Funktionselements.

Das Expansionshilfselement kann wenigstens ein radial expandierbares rohrförmiges Element und/oder wenigstens eine radial expandierbare Schraubenzugfeder aufweisen. Das rohrförmige Element kann zumindest in einem mittleren Abschnitt radial expandierbar sein. Um die Expandierbarkeit des rohrförmigen Elements zu gewährleisten, kann das rohrförmige Element strukturiert sein, insbesondere Längsschlitze und/oder spiralförmige Schlitze aufweisen. Das Expansionshilfselement kann auch eine radial expandierbare Schraubenzugfeder aufweisen. Dabei weist die Schraubenzugfeder im komprimierten Zustand eine größere Steigung der einzelnen Schraubenwindungen auf als im expandierten Zustand. Bei der Entlassung der Schraubenzugfeder aus dem Katheter verkürzt sich diese, wodurch sich der Durchmesser der Schraubenzugfeder erhöht.

In einer weiteren bevorzugten Ausführungsform ist das Expansionshilfselement einteilig mit dem Betätigungselement ausgebildet. Das vereinfacht die Konstruktion des erfindungsgemäßen Zuführsystems signifikant. Insbesondere kann das Betätigungselement einen distalen Endabschnitt aufweisen, der im Wesentliche schraubenzugfederartig geformt ist und somit das Expansionshilfselement bildet.

In einer bevorzugten Variante des Zuführsystems ist im Aufnahmebereich des Betätigungselements ein Halteelement angeordnet. Das Halteelement kann mit dem Betätigungselement fest verbunden sein. Ferner kann vorgesehen sein, dass das Halteelement mit einem proximalen Ende des Funktionselements lösbar verbindbar ist. Konkret kann das Halteelement angepasst sein, eine temporäre Verbindung mit dem Funktionselement einzugehen. Das Halteelement ermöglicht eine zeitweise Fixierung des Funktionselements, so dass das Funktionselement während des Vorschiebens durch den Katheter fest am Betätigungselement angeordnet ist. Die Verbindung zwischen dem Halteelement und dem Funktionselement kann reibschlüssig oder formschlüssig erfolgen. Eine reibschlüssige Verbindung kann auch zwischen dem Funktionselement und dem Expansionshilfselement bestehen, so dass das Expansionshilfselement gleichwohl das Halteelement bilden kann.

Das Halteelement kann auch proximal des Expansionshilfselements angeordnet sein. In diesem Fall bilden das Halteelement und das Expansionshilfselement separate Bauteile. Durch die proximale Anordnung des Halteelements bezogen auf das Expansionshilfselement ist sichergestellt, dass das Funktionselement sich über das Expansionshilfselement erstreckt, wenn ein proximales Ende des Funktionselements mit dem Halteelement verbunden ist. Ein weiterer Vorteil des proximal zum Expansionshilfselement angeordneten Halteelements besteht darin, dass das mit dem Halteelement verbundene Funktionselement wieder in den Katheter zurückziehbar ist, selbst wenn das Expansionshilfselement bereits vollständig aus dem Katheter entlassen und somit vollständig expandiert ist. In diesem Fall ist das proximale Ende des Funktionselements weiterhin innerhalb des Katheters mit dem Halteelement verbunden, so dass der Katheter ohne Weiteres wieder über das Funktionselement geschoben werden kann. Dabei wird das Funktionselement in den komprimierten Zustand zurückgedrängt. Dies ermöglicht eine einfache und gefäßwandschonende Repositionierung des Funktionselements.

Gemäß einem nebengeordneten Aspekt beruht die Erfindung auf dem Gedanken, ein Set mit einem zuvor erläuterten Zuführsystem und einem medizinischen Funktionselement anzugeben, das innerhalb des Katheters im Aufnahmebereich des Betätigungselements angeordnet und mit dem Betätigungselement lösbar verbunden, insbesondere reibverbunden, ist. Dabei ist das Expansionshilfselement vorzugsweise radial innerhalb des Funktionselements angeordnet.

Ein weiterer nebengeordneter Aspekt der Erfindung betrifft ein Verfahren zum Entlassen eines Funktionselements aus einem zuvor erläuterten Zuführsystem. Das erfindungsgemäße Verfahren kann die folgenden Schritte umfassen:
- Das im Aufnahmebereich angeordnete medizinische Funktionselement wird mit dem Betätigungselement durch den Katheter geführt;
- das radial innerhalb des Funktionselement angeordnete Expansionshilfselement wird durch eine längsaxiale Relativbewegung zwischen dem Katheter und dem Betätigungselement sukzessive entlassen, wobei das radial vorgespannte Expansionshilfselement selbsttätig radial expandiert und eine radiale Expansionshilfskraft auf eine Innenumfangsfläche des Funktionselements ausübt, so dass eine radiale Aufweitung des Funktionselements unterstützt wird.

Die Erfindung wird im Folgenden anhand von Ausführungsbeispielen unter Bezugnahme auf die beigefügten, schematischen Zeichnungen näher erläutert. Darin zeigen
- Fig. 1: eine Seitenansicht des erfindungsgemäßen Zuführsystems nach einem bevorzugten Ausführungsbeispiel während des Freisetzens eines medizinischen Funktionselements in einem gekrümmten Gefäßabschnitt;
- Fig. 2a-2c: jeweils eine Seitenansicht des Zuführsystems gemäß Fig. 1 in unterschiedlichen Phasen des Freisetzens des Funktionselements;
- Fig. 3a: eine Seitenansicht eines erfindungsgemäß Zuführsystems nach einem weiteren bevorzugten Ausführungsbeispiel, wobei das Expansionshilfselement als Schraubenzugfeder ausgebildet ist;
- Fig. 3b: eine Seitenansicht des Zuführsystems gemäß Fig. 3a nach Entlassung des Expansionshilfselements aus dem Katheter;
- Fig. 4: eine Seitenansicht eines erfindungsgemäßen Zuführsystems nach einem weiteren bevorzugten Ausführungsbeispiel, wobei das Expansionshilfselement mittels einer Hülse mit dem Betätigungselement gekoppelt ist;
- Fig. 5: eine Seitenansicht eines erfindungsgemäßen Zuführsystems nach einem weiteren bevorzugten Ausführungsbeispiel, wobei das Expansionshilfselement als Fortsetzung eines mit dem Betätigungselement verbundenen Coils ausgebildet ist; und
- Fig. 6: eine Seitenansicht eines erfindungsgemäßen Zuführsystems nach einem weiteren bevorzugten Ausführungsbeispiel, wobei das Expansionshilfselement einteilig mit dem Betätigungselement ausgebildet ist.

Im Allgemeinen ist das erfindungsgemäße Zuführsystem für die Zuführung von medizinischen Funktionselementen im Rahmen von minimalinvasiven medizinischen Eingriffen geeignet. Als zuzuführende Funktionselemente kommen insbesondere Stents, aber auch Blutfilter, Thrombenfänger oder dergleichen selbstexpandierbare medizinische Implantate in Betracht. Vorzugsweise weist das Funktionselement eine Gitterstruktur auf, die aus geflochtenen Drähten oder durch ein entsprechend geschnittenes Vollrohr gebildet ist.

Das Zuführsystem umfasst einen Katheter 10, der in ein Körperhohlorgan, insbesondere ein Blutgefäß 30, einführbar ist. Innerhalb des Katheters 10 ist ein Betätigungselement 20 angeordnet, das im Katheter 10 längsverschiebbar ist. Das Betätigungselement 20 ist vorzugsweise durch einen Führungsdraht gebildet. In einem distalen Endabschnitt 21 des Betätigungselements 20 ist ein Aufnahmebereich 40 für das Funktionselement 50 bzw. einen Stent angeordnet. Der Aufnahmebereich 40 ist vorzugsweise angepasst, um das Funktionselement 50 aufzunehmen und zumindest temporär mit dem Betätigungselement 20 zu verbinden.

Im Aufnahmebereich 40 ist ein Expansionshilfselement 41 angeordnet. Das Expansionshilfselement 41 ist selbstexpandierbar. Insbesondere weist das Expansionshilfselement 41 eine Expansionshilfskraft auf, die radial nach außen wirkt. Das Expansionshilfselement 41 ist also innerhalb des Katheters 10 radial vorgespannt bzw. radial komprimiert.

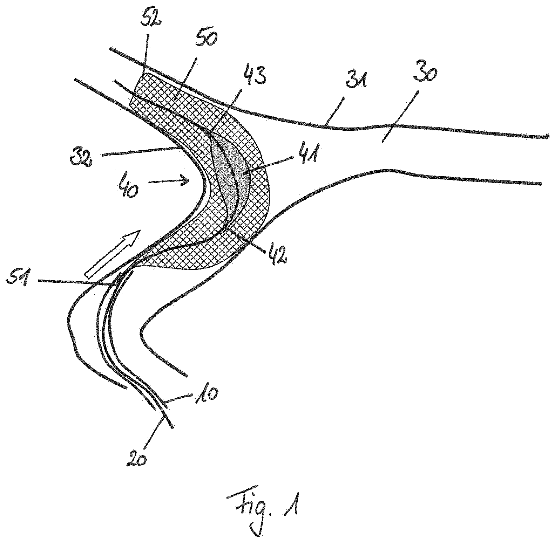

In Fig. 1 ist das Zuführsystem beim Einsetzen eines Funktionselements 50, insbesondere eines Stents, in einen gekrümmten Abschnitt 32 eines Blutgefäßes 30 gezeigt. In der Darstellung gemäß Fig. 1 ist das Funktionselement 50 bereits teilweise aus dem Katheter 10 entlassen und expandiert. Die Entlassung des Funktionselements 50 erfolgt vorzugsweise dadurch, dass der Katheter 10 mit dem darin angeordneten Betätigungselement 20 und dem Funktionselement 50 durch das Blutgefäß 30 bis hinter den gekrümmten Abschnitt 32 geführt wird. Anschließend wird das Betätigungselement 20 ortsfest gehalten und der Katheter 10 in proximale Richtung zurückgezogen. Dadurch wird zunächst das distale Ende 52 des Funktionselements 50 aus dem Katheter 10 entlassen. Das distale Ende 52 des Funktionselements 50 expandiert unmittelbar nach der Freisetzung aus dem Katheter 10. Durch weiteres Zurückziehen des Katheters 10 wird das Funktionselement 50 sukzessive bzw. abschnittsweise freigesetzt und expandiert. In geraden Abschnitten des Blutgefäßes 30 expandiert das Funktionselement 50 gleichmäßig. Das Funktionselement 50 weitet sich also in alle radiale Richtungen gleichmäßig aus. Diese Ausweitung wird lediglich durch die Geometrie der Gefäßwand 31 begrenzt.

Im gekrümmten Abschnitt 32 des Blutgefäßes 30 tendiert das Funktionselement 50 jedoch dazu, sich nicht gleichmäßig radial aufzuweiten, sondern eine ovale Form einzunehmen. Im Extremfall kollabiert das Funktionselement 50 im gekrümmten Abschnitt 32 vollständig. Um dies zu verhindern, ist bei dem erfindungsgemäßen Zuführsystem das Expansionshilfselement 41 vorgesehen, das im Aufnahmebereich 40 angeordnet ist. Konkret ist das Expansionshilfselement 41 in demselben Bereich des Betätigungselements 20 angeordnet, das auch das Funktionselement 50 trägt. Das Expansionshilfselement 41 ist insbesondere radial innerhalb des Funktionselements 50 angeordnet. Die Freilassung und Aufweitung des Expansionshilfselements 41 erfolgt im Wesentlichen analog zur Freilassung des Funktionselements 50. Durch das Zurückziehen des Katheters 10 wird das Expansionshilfselement 41 von seinem distalen Ende 43 zu seinem proximalen Ende 42 sukzessive aus dem Katheter 10 entlassen und expandiert selbsttätig. Dabei übt das Expansionshilfselement 41 eine Expansionshilfskraft auf das Funktionselement 50 aus, so dass eine gleichmäßige Aufweitung des Funktionselements 50, insbesondere auch in gekrümmten Abschnitten 32 des Blutgefäßes 30, unterstützt wird. Die für die Aufweitung des Funktionselements 50 erforderliche Expansionskraft wird also zeitweise, nämlich solange das Expansionshilfselement 41 innerhalb des Funktionselements 50 angeordnet ist, um den Betrag der Expansionshilfskraft des Expansionshilfselements 41 erhöht. Die Expansionshilfskraft des Expansionshilfselements 41 wird vollständig wirksam, wenn das Expansionshilfselement 41, wie in Fig. 1 dargestellt ist, vollständig aus dem Katheter 10 entlassen ist.

Bei der Darstellung gemäß Fig. 1 ist außerdem erkennbar, dass das Funktionselement 50 teilweise entlassen und teilweise, nämlich mit seinem proximalen Ende 51, innerhalb des Katheters 10 angeordnet ist. In diesen Zustand ist das Funktionselement 50 in den Katheter 10 wieder einziehbar. Dies kann zweckmäßig sein, um das Funktionselement 50 beispielsweise in eine andere Position zu bringen. Ferner kann, da das Expansionshilfselement 41 bereits voll expandiert und wirksam ist, das Funktionselement 50 wieder eingezogen werden, wenn sich herausstellen sollte, dass auch die Expansionshilfskraft nicht ausreicht, um ein Einknicken des Funktionselements 50 zu verhindern.

In Fig. 1 ist ferner erkennbar dass das Expansionshilfselement 41 an seinem proximalen Ende 42 mit dem Betätigungselement 20 verbunden ist. Das proximale Ende 42 des Expansionshilfselements 41 ist also am Betätigungselement 20 festgelegt, so dass das proximale Ende 42 an sich nicht expandiert. Somit ergibt sich im expandierten Zustand des Expansionshilfselements 41 eine im Wesentlichen trichterförmige bzw. kegelförmige proximale Spitze des Expansionshilfselements 41. Dieses im Wesentlichen spitz zulaufende proximale Ende 42 des Expansionshilfselements 41 ermöglicht es, dass auch das Expansionshilfselement 41 in den Katheter 10 zurückziehbar ist. Indem der Katheter 10 über das proximale Ende 42 des expandierten Expansionshilfselements 41 geführt wird, wird das Expansionshilfselement 41 in den komprimierten Zustand zurückgedrängt und kann so wieder innerhalb des Katheters 10 angeordnet werden. Auf diese Weise kann das Expansionshilfselement 41 nach vollständiger Freisetzung des Funktionselements 50, insbesondere eines Stents, über den Katheter 10 aus dem Blutgefäß 30 entfernt werden.

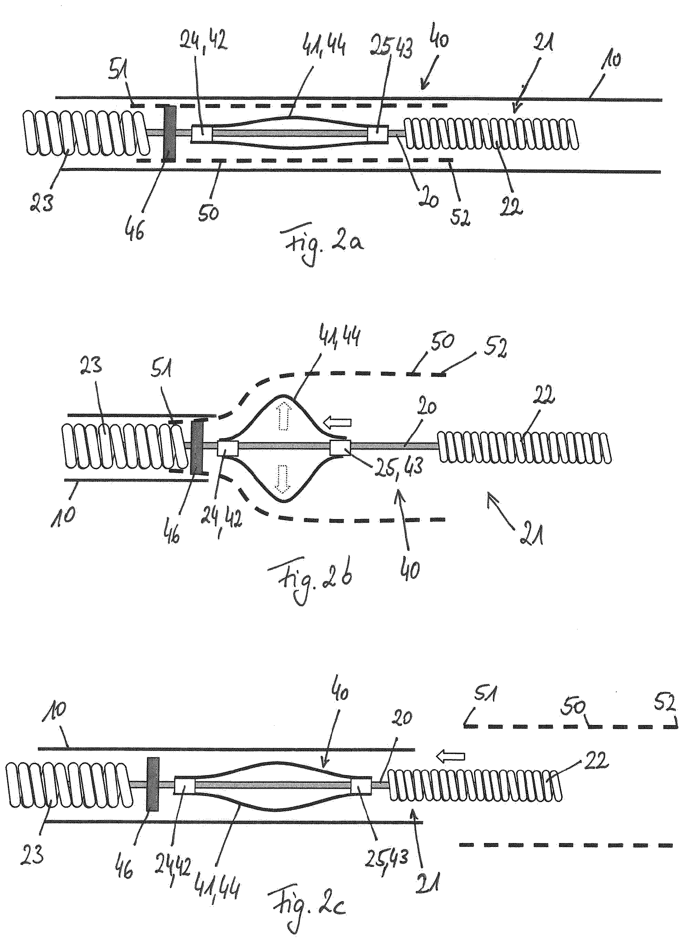

Die Funktionsweise des Expansionshilfselements 41 wird aus den Fig. 2a bis 2c ersichtlich. Fig. 2a zeigt das Zuführsystem mit dem Katheter 10 und dem Betätigungselement 20, insbesondere dem distalen Endabschnitt 21 des Betätigungselements 20. Das Betätigungselement 20 weist im distalen Endabschnitt 21 einen distalen Coil 22 auf, der das Betätigungselement 20 distal begrenzt bzw. eine Spitze des Betätigungselements 20 bildet. Ferner ist ein proximaler Coil 23 am Betätigungselement 20 angeordnet, der beabstandet vom distalen Coil 22 angeordnet ist. Der proximale Coil 23 kann sich bis zu einem proximalen Ende des Betätigungselements 20 erstrecken. Der proximale Coil 23 und der distale Coil 22 sorgen für eine verbesserte Biegeflexibilität des Betätigungselements 20. So ist ermöglicht, dass das Betätigungselement 20, insbesondere der Führungsdraht, gut durch gekrümmte Gefäßabschnitte geführt werden kann.

Zwischen dem distalen Coil 20 und dem proximalen Coil 23 ist der Aufnahmebereich 40 angeordnet. Dabei kann sich der Aufnahmebereich 40 teilweise über den distalen Coil 22 und/oder den proximalen Coil 23 erstrecken. Bei dem Ausführungsbeispiel gemäß den Fig. 2a-2c ist im Aufnahmebereich 40 ein Halteelement 46 angeordnet. Das Halteelement 46 ist distal des proximalen Coils 23 fest mit dem Betätigungselement 20 verbunden. Das Halteelement 46 ist angepasst, das Funktionselement 50 zu halten. Insbesondere kann das Halteelement 46 einen Querschnittsdurchmesser aufweisen, der dem Innendurchmesser des Katheters 10 abzüglich der Wandstärke des Funktionselements 50 entspricht. Somit kann das Halteelement 46 das Funktionselement 50 im Wesentlichen reibschlüssig zwischen dem Katheter 10 und dem Betätigungselement 20 fixieren. Dabei ist das Halteelement 46 vorzugsweise derart angepasst, dass zwischen dem Halteelement 46 und dem Funktionselement 50 eine größere Reibungskraft besteht als zwischen dem Funktionselement 50 und der Katheterinnenwand. Somit ist sichergestellt, dass das Funktionselement 50 gemeinsam mit dem Betätigungselement 20 im Katheter 10 verschiebbar ist. Alternativ kann die Verbindung zwischen dem Funktionselement 50 und dem Halteelement 46 auch formschlüssig erfolgen. Beispielsweise kann das Halteelement 46 hakenartige Elemente aufweisen, die in das Funktionselement 50, beispielsweise Maschen oder Zellen einer Gitterstruktur des Funktionselements 50 eingreifen.

Das Halteelement 46 ist proximal zum Expansionshilfselement 41 angeordnet. Das Expansionshilfselement ist bei dem Ausführungsbeispiel gemäß Fig. 2a-2c als radial expandierbares rohrförmiges Element ausgebildet. Das rohrförmige Element kann Längsschlitze aufweisen, so dass das rohrförmige Element zumindest in einem mittleren Bereich radial expandierbar ist. Alternativ oder zusätzlich kann das rohrförmige Element 44 spiralförmige Schlitze umfassen.

Das Expansionshilfselement 41 weist ein proximales Ende 42 auf, das mit einer proximalen Hülse 24 mit dem Betätigungselement 20 fest verbunden ist. Das distale Ende 43 des Expansionshilfselements 41 ist mit einer distalen Hülse 25 fest verbunden. Die distale Hülse 25 ist längsverschieblich auf dem Betätigungselement 20 gelagert. Somit ist gewährleistet, dass der Abstand zwischen der proximalen Hülse 24 und der distalen Hülse 25 variierbar ist.

Das proximale Ende 42 des Expansionshilfselements 41 kann auch mit der proximalen Hülse 24 oder mit dem Betätigungselement 20 direkt verklebt oder schweißverbunden sein.

Innerhalb des Katheters 10 ist das Expansionshilfselement 41 gestreckt (Fig. 2a). Bei Freilassung des Funktionselements 50 aus dem Katheter 10 expandiert das Funktionselement 50. Die Expansion erfolgt dabei sukzessive vom distalen Ende 52 in Richtung zum proximalen Ende 51 des Funktionselements 50. Sobald das Expansionshilfselement 41 aus dem Katheter 10 entlassen wird, erfolgt ebenfalls eine Expansion des Expansionshilfselements 41, die am distalen Ende 43 beginnt und am proximalen Ende 42 des Expansionshilfselements 41 endet. Wie in Fig. 2b erkennbar ist, bewirkt die selbsttätige Aufweitung des Expansionshilfselements 41 gleichzeitig eine axiale Bewegung der distalen Hülse 25 in Richtung zur proximalen Hülse 24. Der Abstand zwischen der distalen Hülse 25 und der proximalen Hülse 24 verringert sich. Somit wird im Wesentlichen die distale Hülse 25 der radialen Expansion des Expansionshilfselements 41 nachgeführt.

Wie in Fig. 2b anhand der gepunkteten Pfeile angedeutet ist, weist das Expansionshilfselement 41 eine Expansionshilfskraft auf, die in radiale Richtung nach au-ßen wirkt. Die Expansionshilfskraft wirkt insbesondere auf eine Innenfläche des Funktionselements 50. Somit wird die Expansion des Funktionselements 50 wirksam unterstützt. So kann ein Kollabieren des Funktionselements 50 beim Freisetzen in gekrümmten Abschnitten 32 eines Blutgefäßes 30 vermieden werden.

Sobald das Funktionselement 50 vollständig aus dem Katheter 10 entlassen und im Blutgefäß 30 aufgespannt ist, wird das Betätigungselement 20 in proximale Richtung gezogen, wobei der Katheter 10 ortsfest gehalten wird. Das Betätigungselement 20 wird also in den Katheter zurückgezogen. Dabei wird das Expansionshilfselement 41 zwangskomprimiert und gleitet in den Katheter 10 zurück. Sobald das Expansionshilfselement 41 vollständig im Katheter 10 angeordnet ist, kann dieser aus dem Blutgefäß 30 entfernt werden. Da das Expansionshilfselement 41 nun innerhalb des Katheters 10 komprimiert ist, wird verhindert, dass beim Entfernen des Expansionshilfselements 41 aus dem Blutgefäß 30 Gefäßwände beschädigt werden.

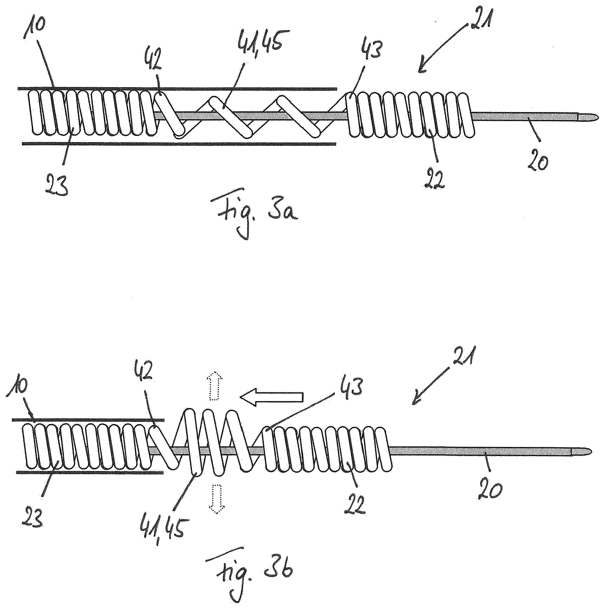

Ein alternatives Ausführungsbeispiel für das Expansionshilfselement 41 zeigen die Fig. 3a und 3b. Darin ist ebenfalls ein Katheter 10 gezeigt, in dem ein Betätigungselement 20 längsverschieblich angeordnet ist. Das Betätigungselement 20 weist in einem distalen Endabschnitt 21 einen distalen Coil 22 auf. In diesem Fall erstreckt sich der distale Coil 22 jedoch nicht bis zur Spitze des Betätigungselements 20. Vielmehr ist die Spitze des Betätigungselements 20 coilfrei ausgebildet. Ferner ist ein proximaler Coil 23 vorgesehen, der vom distalen Coil 22 beabstandet angeordnet ist.

Zwischen dem proximalen Coil 23 und dem distalen Coil 22 ist ein Expansionshilfselement 41 in Form einer Schraubenzugfeder 45 angeordnet. Die Schraubenzugfeder 45 ist insbesondere einteilig mit dem distalen Coil 22 und dem proximalen Coil 23 ausgebildet. Der distale Coil 22 und der proximale Coil 23 weisen im Unterschied zum Expansionshilfselement 41 keine Federkraftkomponente auf. Innerhalb des Katheters 10 ist die Schraubenzugfeder 45 in einem gestreckten Zustand. Grundsätzlich ist vorgesehen, dass die Schraubenzugfeder 45 in einem entspannten, also vollständig expandierten, Zustand einen Querschnittsdurchmesser aufweist, der größer als der Innendurchmesser des Katheters 10 ist. Bei Anordnung der Schraubenzugfeder 45 innerhalb des Katheters 10 wird die Schraubenzugfeder 45 folglich gestreckt, wie in Fig. 3a erkennbar ist. Sobald die Schraubenzugfeder 45 den Katheter 10 verlassen hat, tendiert die Schraubenzugfeder 45 dazu, ihren Ruhezustand wieder einzunehmen. Infolgedessen verkürzt sich die Schraubenzugfeder 45. Dadurch nehmen die einzelnen Windungen der Schraubenzugfeder 45 ihren Ruhezustandsdurchmesser ein. Die Schraubenzugfeder 45 expandiert. Die dabei wirkende Expansionshilfskraft unterstützt das in den Fig. 3a und 3b nicht dargestellte Funktionselement 50 bei der Expansion in einem Blutgefäß 30.

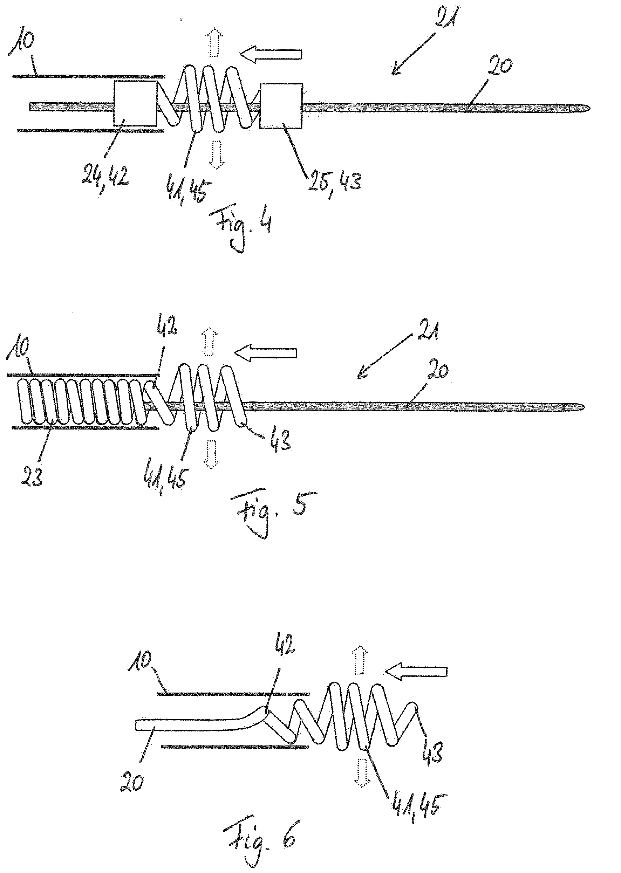

In den Fig. 4-6 sind unterschiedliche Varianten für die Verbindung zwischen dem Betätigungselement 20 und dem Expansionshilfselement 41, insbesondere einer Schraubenzugfeder 45, gezeigt. Gemäß Fig. 4 kann vorgesehen sein, dass die Schraubenzugfeder 45 an den Längsenden mit jeweils einer Hülse fest verbunden ist. Insbesondere kann die Schraubenzugfeder 45 eine proximale Hülse 24 und eine distale Hülse 25 aufweisen. Die proximale Hülse 24 ist vorzugsweise fest mit dem Betätigungselement 20 gekoppelt. Die distale Hülse 25 ist hingegen auf dem Betätigungselement 20 längsverschieblich angeordnet. Bei der Expansion und der gleichzeitigen Verkürzung der Schraubenzugfeder 45 gleitet also die distale Hülse 25 entlang des Betätigungselements 20. Die distale Hülse 25 nähert sich dabei der proximalen Hülse 24 an.

Alternativ kann die Schraubenzugfeder 45 proximal einteilig mit einem proximalen Coil 23 des Betätigungselements 20 verbunden sein. Das distale Ende 43 des Expansionshilfselements 41 bzw. der Schraubenzugfeder 45 kann hingegen frei auf dem Betätigungselement 20 angeordnet sein. Somit ist das distale Ende 43 frei entlang des Betätigungselements bewegbar, wodurch die bei der Expansion der Schraubenzugfeder 45 auftretende Verkürzung der Schraubenzugfeder 45 ermöglicht ist.

Eine konstruktiv besonders einfache Variante ist in Fig. 6 dargestellt. Dabei ist das Expansionshilfselement 41, das hier als Schraubenzugfeder 45 ausgebildet ist, stoffschlüssig, insbesondere einteilig, mit dem Betätigungselement 20 gekoppelt. Konkret weist das Betätigungselement 20 eine schraubenzugfederförmige Spitze auf bzw. der distale Endabschnitt 21 des Betätigungselements 20 als Schraubenzugfeder 45 geformt. Das hat den Vorteil, dass das Betätigungselement 20 ist insgesamt, insbesondere im gestreckten Zustand der Schraubenzugfeder 45 bzw. des distalen Endabschnitts 21, einen relativ kleinen Querschnittsdurchmesser aufweist. Somit eignet sich diese Variante besonders zur Zuführung eines Funktionselements 50 in kleine Blutgefäße 30.

Der Effekt, dass sich das Expansionshilfselement 41 bei der Radialexpansion verkürzt, hat den zusätzlichen Vorteil, dass das Expansionshilfselement 41 so bei der Entlassung aus dem Katheter 10 schrittweise entlang des aufzuweitenden Funktionselements 50 gleitet bzw. wandert und somit einen relativ größeren Abschnitt des Funktionselements 50 bei der Expansion unterstützt.

Generell kann das Expansionshilfselement 41 als Schraubenzugfeder 45 oder als rohrförmiges Element 44 mit einem Drahtgeflecht ausgebildet sein. Das Expansionselement 41 kann helixförmig ausgebildet sein, insbesondere durch ein helixförmig um eine Längsachse gewundenes Element gebildet sein. Ferner kann das Expansionselement 41 als radial expandierbares, rohrförmiges Element 44 ausgebildet sein, das, beispielsweise durch Laserschneiden, aus einem Vollrohr hergestellt oder durch ein Geflecht aus sich kreuzenden Drähten gebildet ist. Es hat sich als vorteilhaft herausgestellt, wenn ein bestimmter Steigungswinkel der Schraubenzugfeder 45 bzw. ein bestimmter Flechtwinkel des Drahtgeflechts eingestellt wird. Dieser Winkel beträgt vorzugsweise wenigstens 30°, insbesondere wenigstens 40°, insbesondere wenigstens 45°, insbesondere wenigstens 50°, insbesondere wenigstens 55°, insbesondere wenigstens 60°, insbesondere wenigstens 75°, insbesondere wenigstens 80°. Konkret wird durch Einstellung dieses Winkels mit den genannten Werten die Radialkraft des Expansionshilfselements 41 erhöht. Gleichzeitig wird mit den vorgeschlagenen Winkeln erreicht, dass sich das Expansionshilfselement 41 relativ stark verkürzt und somit über einen vergleichsweise großen Abschnitt des Funktionselements 50 die Expansionshilfskraft aufbringt.

Die Schraubenzugfeder 45 ist vorzugsweise durch ein Drahtelement gebildet. Das Drahtelement der Schraubenzugfeder 45 bzw. die Drahtelemente des Gittergeflechts des rohrförmigen Elements 44 weisen vorzugsweise einen Querschnittsdurchmesser von wenigstens 50 µm, insbesondere wenigstens 75 µm, insbesondere wenigstens 85 µm, insbesondere wenigstens 100 µm, insbesondere wenigstens 120 µm, insbesondere wenigstens 150 µm, insbesondere wenigstens 200 µm, auf.

Es ist möglich, dass das Expansionshilfselement 41 im vollständig expandierten Zustand einen kleineren Querschnittsdurchmesser aufweist als das Funktionselement 50 im vollständig expandierten Zustand. Wesentlich ist, dass das Expansionshilfselement 41 das Funktionselement 50 zumindest in einer Anfangsphase der Expansion unterstützt. Vorzugsweise weist das Expansionshilfselement 41 im vollständig expandierten Zustand einen Querschnittsdurchmesser von höchstens 4 mm, insbesondere höchstens 3,5 mm, insbesondere höchstens 3 mm, insbesondere höchstens 2,5 mm, insbesondere höchstens 2 mm, insbesondere höchstens 1,5 mm, insbesondere höchstens 1 mm, auf. Diese Werte beziehen sich auf den Au-ßendurchmesser des Expansionshilfselements 41.

Das Verhältnis zwischen dem Außendurchmesser des Expansionshilfselements 41 im vollständig expandierten Zustand und dem Außendurchmesser des Funktionselements 50 im vollständig expandierten Zustand kann höchstens 0,8, insbesondere höchstens 0,7, insbesondere höchstens 0,6, insbesondere höchstens 0,5, insbesondere höchstens 0,4, insbesondere höchstens 0,3, insbesondere höchstens 0,2, insbesondere höchstens 0,1, betragen. Auf diese Weise wird sicher vermieden, dass das Expansionshilfselement 41 entlang der Innenfläche des Funktionselements 50 reibt, wenn das Funktionselement 50 bereits vollständig freigesetzt und das Expansionshilfselement 41 in den Katheter 10 zurückgezogen wird.

Hinsichtlich der Expansionshilfskraft ist vorteilhaft vorgesehen, dass das Expansionshilfselement 41 eine Expansionshilfskraft von wenigstens 30 mm Hg, insbesondere wenigstens 50 mm Hg, insbesondere wenigstens 100 mm Hg, insbesondere wenigstens 200 mm Hg, aufweist, wenn das Expansionshilfselement 41 ausgehend vom vollständig expandierten Zustand um 0,5 mm im Querschnitt komprimiert ist. Die vorgenannten Werte für die Expansionshilfskraft bestehen also in einem expandierten Zustand des Expansionshilfselements 41, bei dem Expansionshilfselement 41 einen Querschnittsdurchmesser aufweist, der um 0,5 mm kleiner ist als der Querschnittsdurchmesser des Expansionshilfselements 41 im vollständig expandierten, d.h. kraftunbelasteten,Zustand.

Die Expansionshilfskraft des Expansionshilfselements 41 kann derart eingestellt sein, dass zwischen der Radialkraft des Funktionselements 50 und der Expansionshilfskraft, die das Expansionshilfselement 41 aufbringt, ein Verhältnis eingestellt ist, das höchstens 0,8, insbesondere höchstens 0,6, insbesondere höchstens 0,4, insbesondere höchstens 0,2, beträgt.

Generell kann das Expansionshilfselement 41 im komprimierten bzw. gestreckten Zustand innerhalb des Katheters 10 länger oder kürzer als das in Katheter 10 angeordnete Funktionselement 50 sein. Das Verhältnis des zwischen der Länge des in Katheter 10 gestreckten Expansionshilfselements 41 und des in Katheter 10 angeordneten Funktionselements 50 kann höchstens 0,8, insbesondere höchstens 0,6, insbesondere höchstens 0,4, insbesondere höchstens 0,2, betragen. Umgekehrt kann das Verhältnis der Länge des in Katheter 10 angeordneten Funktionselements 50 und der Länge des in Katheter 10 gestreckten Expansionshilfselements 41 ebenfalls höchstens 0,8, insbesondere höchstens 0,6, insbesondere höchstens 0,4, insbesondere höchstens 0,2, betragen.

### Bezuaszeichenliste

- 10: Katheter
- 20: Betätigungselement
- 21: distaler Endabschnitt
- 22: distaler Coil
- 23: proximaler Coil
- 24: proximale Hülse
- 25: distale Hülse
- 30: Blutgefäß
- 31: Gefäßwand
- 32: gekrümmter Abschnitt
- 40: Aufnahmebereich
- 41: Expansionshilfselement
- 42: proximales Ende des Expansionshilfselements 41
- 43: distales Ende des Expansionshilfselements 41
- 44: rohrförmiges Element
- 45: schraubenzugfeder
- 46: Halteelement
- 50: Funktionselement
- 51: proximales Ende des Funktionselements 50
- 52: distales Ende des Funktionselements 50

## Patentansprüche

1. Zuführsystem für ein medizinisches Funktionselement (50) mit einem Katheter (10) und einem Betätigungselement (20), das im Katheter (10) längsverschieblich angeordnet ist und in einem distalen Endabschnitt (21) einen Aufnahmebereich (40) für das Funktionselement (50) aufweist, wobei das Betätigungselement (20) mit dem Funktionselement (50) lösbar verbindbar ist derart, dass das Funktionselement (50) im Gebrauch durch das Betätigungselement (20) relativ zum Katheter (10) bewegbar ist, wobei
im Aufnahmebereich (40) ein Expansionshilfselement (41) angeordnet ist, das innerhalb des Katheters (10) radial vorgespannt und nach der Entlassung aus dem Katheter (10) selbsttätig aufweitbar ist, wobei das Expansionshilfselement (41) eine radiale Expansionshilfskraft aufweist derart, dass eine radiale Aufweitung des Funktionselements (50) unterstützbar ist oder unterstützt wird,
**dadurch gekennzeichnet, dass**
das Expansionshilfselement (41) wenigstens ein radial expandierbares rohrförmiges Element (44) mit einem Drahtgeflecht und/oder wenigstens eine radial expandierbare Schraubenzugfeder (45) aufweist.

2. Zuführsystem nach Anspruch 1,
**dadurch gekennzeichnet, dass**
das Expansionshilfselement (41) ein proximales Ende (42) und ein distales Ende (43) aufweist, wobei das proximale Ende (42) fest und das distale Ende (43) längsverschieblich mit dem Betätigungselement (20) verbunden sind.

3. Zuführsystem nach einem der vorhergehenden Ansprüche,
**dadurch gekennzeichnet, dass**
das Expansionshilfselement (41) einteilig mit dem Betätigungselement (20) ausgebildet ist.

4. Zuführsystem nach einem der vorhergehenden Ansprüche,
**dadurch gekennzeichnet, dass**
im Aufnahmebereich (40) ein Halteelement (46) angeordnet ist, das mit dem Betätigungselement (20) fest verbunden und mit einem proximalen Ende (51) des Funktionselements (50) lösbar verbindbar ist.

5. Zuführsystem nach Anspruch 5,
**dadurch gekennzeichnet, dass**
das Halteelement (46) proximal des Expansionshilfselements (41) angeordnet ist.

6. Set mit einem Zuführsystem nach einem der vorhergehenden Ansprüche und mit einem medizinischen Funktionselement (50), das innerhalb des Katheters (10) im Aufnahmebereich (40) des Betätigungselements (20) angeordnet und mit dem Betätigungselement (20) lösbar verbunden, insbesondere reibverbunden, ist, wobei das Expansionshilfselement (41) radial innerhalb des Funktionselements (50) angeordnet ist.

7. Verfahren zum Entlassen eines Funktionselements (50) aus einem Zuführsystem nach einem der Ansprüche 1 bis 6, wobei das Verfahren die folgenden Schritte umfasst:
- das im Aufnahmebereich (40) angeordnete medizinische Funktionselement (50) wird mit dem Betätigungselement (20) durch den Katheter (10) geführt;
- das radial innerhalb des Funktionselements (50) angeordnete Expansionshilfselement (41) wird durch eine längsaxiale Relativbewegung zwischen dem Katheter (10) und dem Betätigungselement (20) sukzessive entlassen, wobei das radial vorgespannte Expansionshilfselement (41) selbsttätig radial expandiert und eine radiale Expansionshilfskraft auf eine Innenumfangsfläche des Funktionselements (50) ausübt derart, dass eine radiale Aufweitung des Funktionselements (50) unterstützt wird.

## Claims

1. A feed system for a medical function element (50), comprising a catheter (10) and an actuating element (20), which is disposed in the catheter (10) so that it is longitudinally displaceable and has a receiving area (40) in a distal end section (21) for the function element (50), wherein the actuating element (20) is releasably connectable to the function element (50), such that the function element (50) is movable by the actuating element (20) relative to the catheter (10) during use, wherein
an expansion assistance element (41) is disposed in the receiving area (40), which is radially prestressed inside the catheter (10) and can be self-expanded after being released from the catheter (10), wherein the expansion assistance element (41) has a radial expansion assistance force, such that a radial expansion of the function element (50) is or can be supported,
**characterized in that**
the expansion assistance element (41) comprises at least one radially expandable tubular element (44) having a wire mesh and/or at least one radially expandable helical tension spring (45).

2. The feed system according to claim 1,
**characterized in that**
the expansion assistance element (41) has a proximal end (42) and a distal end (43), wherein the proximal end (42) is connected fixedly to the actuating element (20), and the distal end (43) is connected to same in a longitudinally displaceable manner.

3. The feed system according to any one of the preceding claims,
**characterized in that**
the expansion assistance element (41) is designed in one piece with the actuating element (20).

4. The feed system according to any one of the preceding claims,
**characterized in that**
a retaining element (46), which is fixedly connected to the actuating element (20) and can be releasably connected to a proximal end (51) of the function element (50), is disposed in the receiving area (40).

5. The feed system according to claim 5,
**characterized in that**
the retaining element (46) is disposed proximally to the expansion assistance element (41).

6. A kit having a feed system according to any one of the preceding claims and having a medical function element (50), which is disposed inside of the catheter (10) in the receiving area (40) of the actuating element (20) and is releasably connected to the actuating element (20), in particular being frictionally connected, wherein the expansion assistance element (41) is disposed radially inside the function element (50).

7. The method for releasing a function element (50) out of a feed system according to any one of claims 1 to 6, wherein the method comprises the following steps:
- the medical function element (50) disposed in the receiving area (40) is guided with the actuating element (20) through the catheter (10);
- the expansion assistance element (41) disposed radially inside the function element (50) is released successively by a longitudinally axial relative movement between the catheter (10) and the actuating element (20), wherein the radially prestressed expansion assistance element (41) self-expands radially and exerts a radial expansion assistance force on an inside circumferential surface of the function element (50), such that a radial expansion of the function element (50) is supported.

## Revendications

1. Système d'acheminement pour élément fonctionnel médical (50) comportant un cathéter (10) et un élément d'actionnement (20) qui est disposé de manière à être déplaçable longitudinalement dans le cathéter (10) et présente, dans une section terminale distale (21), une zone de réception (40) pour l'élément fonctionnel (50), l'élément d'actionnement (20) étant relié de manière dissociable à l'élément fonctionnel (50) de manière à ce que l'élément fonctionnel (50) soit à l'usage mobile grâce à l'élément d'actionnement (20) par rapport au cathéter (10),
étant disposé dans la zone de réception (40) un élément auxiliaire d'expansion (41) qui est précontraint radialement dans le cathéter (10) et est expansible automatiquement une fois extrait du cathéter (10), l'élément auxiliaire d'expansion (41) présentant une force auxiliaire d'expansion telle qu'un élargissement radial de l'élément fonctionnel (50) peut être assistée ou est assistée,
**caractérisé en ce que**
l'élément auxiliaire d'expansion (41) présente au moins un élément tubulaire expansible radialement (44) et comportant un treillis métallique et/ou au moins un ressort de traction hélicoïdal expansible radialement (45).

2. Système d'acheminement selon la revendication 1,
**caractérisé en ce que**
l'élément auxiliaire d'expansion (41) présente une extrémité proximale (42) et une extrémité distale (43), l'extrémité proximale (42) étant reliée fixement à l'extrémité distale (43), de manière à être déplaçable longitudinalement, à l'élément d'actionnement (20).

3. Système d'acheminement selon une des revendications précédentes,
**caractérisé en ce que**
l'élément auxiliaire d'expansion (41) est réalisé en une seule pièce avec l'élément d'actionnement (20).

4. Système d'acheminement selon une des revendications précédentes,
**caractérisé en ce que**,
dans la zone de réception (40), est disposé un élément de retenue (46) qui est relié fixement à l'élément d'actionnement (20) et de manière dissociable à une extrémité proximale (51) de l'élément fonctionnel (50).

5. Système d'acheminement selon la revendication 5,
**caractérisé en ce que**
l'élément de retenue (46) est disposé de manière proximale par rapport à l'élément auxiliaire d'expansion (41).

6. Ensemble comportant un système d'acheminement selon une des revendications précédentes et un élément fonctionnel médical (50) qui est disposé dans le cathéter (10) dans la zone de réception (40) de l'élément d'actionnement (20) et est relié de manière dissociable, en particulier relié par friction, à l'élément d'actionnement (20), l'élément auxiliaire d'expansion (41) étant disposé radialement dans l'élément fonctionnel (50).

7. Procédé d'extraction d'un élément fonctionnel (50) hors d'un système d'acheminement selon une des revendications 1 à 6, ce procédé comprenant les étapes suivantes :
- l'élément fonctionnel médical (50) disposé dans la zone de réception (40) est guidé avec l'élément d'actionnement (20) à travers le cathéter (10) ;
- l'élément auxiliaire d'expansion (41) disposé radialement dans l'élément fonctionnel (50) est extrait successivement par un mouvement relatif axial longitudinal entre le cathéter (10) et l'élément d'actionnement (20), l'élément auxiliaire d'expansion précontraint radialement (41) se dilatant automatiquement radialement et exerçant une force auxiliaire radiale d'expansion sur une surface circonférentielle interne de l'élément fonctionnel (50) de manière à assister une expansion radiale de l'élément fonctionnel (50).
